# EUROPEAN PATENT SPECIFICATION

(11) **EP 2 988 731 B1**
(45) Date of publication and mention of the grant of the patent: **17.02.2021**
(21) Application number: 14788755.8
(22) Date of filing: 23.04.2014
(51) Int. Cl.: A61K 31/23, A61P 17/16, A61P 17/02, A61L 15/00, A61K 47/14, A61K 9/00, A61K 31/14, A61K 45/06, A61K 31/155, A61K 31/19, A61K 33/40, A61K 33/38, A61K 33/18, A61K 31/231, A61K 9/06

(54) **COMPOSITION FOR USE IN REDUCING SCAB FORMATION AND PROMOTING HEALING**
ZUSAMMENSETZUNG ZUR VERRINGERUNG VON KRUSTENBILDUNG UND ZUR FÖRDERUNG DER WUNDHEILUNG
COMPOSITION DESTINÉE À ÊTRE UTILISÉE DANS LA RÉDUCTION DE LA FORMATION DE CROÛTES ET FAVORISANT LA GUÉRISON

(30) Priority: 26.04.2013 SE 1350518
(43) Date of publication of application: 02.03.2016
(73) Proprietor: BIOGLAN AB, S-202 13 Malmö (SE)
(72) Inventor: SVENSSON, Birgitta, S-218 31 Bunkeflostrand (SE); LINSEFORS, Lotta, S-224 67 Lund (SE)
(74) Representative: AWA Sweden AB
(86) International application number: PCT/SE2014/050487
(87) International publication number: WO 2014/175814

(56) References cited:
- WO-A1-82/03173
- SE-B- 346 214
- US-A- 3 772 446
- US-A1- 2007 053 957
- US-A1- 2007 053 957
- BRYAN J: 'Moist wound healing:a concept that changed our practice' JOURNAL OF WOUND CARE vol. 13, no. 6, June 2004, pages 227 - 228, XP008178802
- SKULASON S ET AL.: 'A study of the clinical activity of a gel combining monocaprin and doxycycline:a novel treatment for herpes labialis' JOURNAL OF ORAL PATHOLOGY & MEDICINE vol. 41, 2012, pages 61 - 67, XP055245240

## Description

### Technical Field of the Invention

The present invention relates to a composition comprising at least one monoglyceride chosen from glyceryl monolaurate, glyceryl monomyristate and glyceryl monocaprylate, and at least one solvent, for use in reducing scab formation and promote healing in connection with injuries and wounds in mammals.

### Background of the invention

The concept *moist wound healing* originates from 1962 when George Winter discovered that epithelisation would proceed twice as fast in a moist wound environment as under a dry scab. During wound healing, exuded cells, e.g. white blood cells, and fluid, containing growth factors and enzymes, stimulate healing. Maintaining a moist and warm environment preserves these exudates and allows diffusion of cells and other relevant substances; speeding wound healing and promote skin growth. A moist and warm environment can be maintained by occlusion of the wound. The most common way to achieve a moist and warm environment and to treat acute and chronic wounds are to use some kind of occluding dressing made of polymeric material. Occlusion can also be achieved by use of an ointment or a cream forming an occlusive film. Under the dressing/film, moist in the skin can be build up from below creating a moist and warm environment in the wound.

Complete air and water tight dressings/films have the disadvantage that the bacteria trapped under the dressing/film can reproduce optimally which may lead to increased bacterial proliferation and infection. Presence of bacteria in a wound slows down the healing process. Excess moist in the wound may also damage the surrounding skin, leading to peri-wound maceration and skin breakdown. The skin becomes soft and typically white in colour. The pH also increases under an occlusive dressing/film, which makes the healing process less efficient since for example the protease activity increase. The skin is more prone for bacterial infection also at elevated pH. Occlusive dressings are therefore often combined with hydrogels or other polymers that control the moisture in the wound but the dressing still provide the tight seal to the environment. These absorbant dressings have many advantages when it comes to healing of difficult to heal wounds, but they are expensive and the dressings are not preferred to be used on cuts and wounds in for example the face where they become visible or on extremities where a dressing is difficult to attach. Dressings/films can also be combined with antimicrobial agents such as antibiotics, iodine and silver to decrease the risk for infection. The use of this type of antimicrobial products should be minimised due to the risk for bacterial resistance.

By contrast, dry dressings, such as typical plasters normally used for small cuts and wounds, allow loss of warmth and moisture. When the wound becomes dry and a scab is formed, the wound is more prone to re-injury when exposed to new trauma such as pressure when using the injured extremity in daily life. The diffusion of exuded cells and other relevant substances are decreased under the scab and the healing time is prolonged.

Another problem when using dressings is the great risk that healthy or new tissue is removed upon removal of the dressing. The newly formed skin is interacting with and may even grow into the dressing and then the new skin is removed when the dressing is removed, leading to prolonged healing time.

US2002031556 discloses a topical, pharmaceutical composition containing hydrogen peroxide. The main purpose is to control the stability and effect of hydrogen peroxide in the composition for delivery to the skin.

WO93/20812 discloses an antimicrobial composition of a combination of monoglycerides and a chemical substance such as carbamide or a local anaesthetic of the amide type.

WO82/03173 discloses a germicidal composition consisting of an aqueous suspension of hydrophilic lipid crystals of 1-monolaurin and preferably also 1-monomyristine and hydrogen peroxide. The hydrophilic lipid crystals stabilize the hydrogen peroxide to the effect that the composition retains its germicidal power even after having been stored for a long time.

US3772446 describes an ointment base containing monoglycerides dispersed in the aqueuos solvent of water only, having beneficial properties with regard to consistency and spreading

Reference is also made to US2007/053957.

There is a continuous need to develop better and more safe systems for wound healing and especially reducing scab formation in connection with cuts and wounds.

### Summary of the invention

The present invention relates in one aspect to a composition comprising at least one monoglyceride chosen from glyceryl monolaurate, glyceryl monomyristate and glyceryl monocaprylate, and at least one hydrophilic solvent, having a liquid crystalline lamellar structure at a temperature of above about 37°C, a solid crystalline lamellar structure below about 30°C and in transition from said solid and liquid crystalline lamellar structures at from about 30°C to about 37°C, a humectant, and optionally one or more of a stabilizer, a preservative, an emollient, a buffering agent, a water retaining compound, or an antimicrobial agent, for use in promoting healing in connection with injuries and wounds in mammals, wherein the composition being a cream.

The monoglycerides comprise a mixture of C12 glycerol monolaurate and C14 glycerol monomyristate at a ratio of 1:4 to 4:1 at a total amount 15 to 30 % by weight, and the solvent is present in an amount adding up to 100% by weight.

### Brief Description of the drawings

Figure 1 depicts high resolution florescence microscopic images. Two types of flouroprobes, Alexa dextran and Nile Red, were used in order to stain the hydrophilic areas (green colour) and hydrophobic areas (red colour), respectively. Solid crystalline particles are dispersed in a continuous phase below 30°C. The left hand figure shows the wet film structure after addition of the Alexa dextran probe at 25°C. The dispersed lipid crystals are dark. The right hand picture shows the wet film structure after addition of the Nile Red probe at 33ºC. It can be observed that the crystals melt.
Figure 2 depicts the mean percent rate of re-epithelisation with time from wounding in humans.
Figure 3 depicts the mean percent epidermal resurfacing with time from wounding in pigs.
Figure 4 depicts the results of the evaporation experiment 5.

### Detailed Description of the invention

The present invention is in accordance with the appended claims. The compositions of this invention form a semi-occlusive moisturising protective film in the wound during the healing process by reducing scab formation.

The use of the wound healing composition of the present invention instead of a protective dressing or in combination with a protective dressing, decrease the risk for disturbance of the wound healing process at redressing. The composition forms a soft protective film between the dressing and the wound surface. Without a dressing the composition will form a soft protective film to the air. The film is easily removed with water when wanted. The wound will be less sensitive to mechanical trauma and the film will help to keep the body temperature. Mechanical trauma in the present context means rupture of the new skin that has formed during the wound healing. The composition will control the amount of moist in the wound. The composition will control the moist during the whole healing process; from the phase when the bleeding has stopped in an acute wound, a situation with excess moist, until the wound is closed and the scar building phase starts, with shortage of moist, which is the main process in wound healing.

The presence of humectants provide a moisturising effect and will improve the skin condition of the new skin formed during healing.

The low pH of the composition will obtain the normal skin pH, facilitate healing, and decrease the risk for infection. An antimicrobial compound, such as hydrogen peroxide, can be added to further decrease the risk for infection and promote healing.

The compositions according to the present invention are suitable to be used in cuts and wounds, in mammals; cuts and wounds that are acute or chronic; cuts and wounds that are drying unless protected. Wounds that are left to dry in air will create scabs. If a scab is formed, it is more difficult for the wound to close itself. This since under dry conditions new skin tissue will have a tough time to form due to low migration capacity of cells and other relevant substances. Said cuts and wounds could for instance be abrasions, cuts, burns, lacerations, blisters, acne spots, bites, rash wounds, chronic wounds including venous and diabetic leg and foot ulcers, ulcers caused by poor blood circulation.

In a disclosure of the invention the composition comprises additionally at least one of a humectant, an emollient, a buffering agent, a preservative, a water retaining compound, an antimicrobial agent or a stabilizer.

The solvent is chosen from the group *hydrophilic solvents* such as water, glycerol, propylene glycol, butylene glycol, dipropylene glycol, propanediol, and propylene carbonate. Some low molecular weight solvents such as ethanol, isopropyl alcohol and acetone increase the solubility of the monoglycerides and destroy the crystal structure. The at least one humectant is, in one embodiment, chosen from the group consisting of glycerol, propylene glycol, butylene glycol, hexylene glycol, triacetin, panthenol, pidolic acid, alfa-hydroxy acids, sorbitol, xylitol, manitol, vitamin B3 and urea. A humectants is any one of a group of hygroscopic substances used to keep skin moist. They are characterized by containing hydroxyl groups, amines or carboxyl groups and are sometimes esterified. Some solvents are categorized as humectants, for example glycerol, propylene glycol and butylene glycol. In an embodiment of this invention the solvent, is preferably at least partly chosen from the humectants, to result in a more moisturising composition. The at least one emollient is, in another embodiment, chosen from the group consisting of fat and vegetable oil, including monoglycerides, wax, petrolatum, hard or soft paraffin, and mineral oil. In another embodiment the water retaining compound is chosen from the group consisting of surfactants and polymers, cellulose based polymers, polyacrylic acid derivatives, polysaccharides, non-ionic surfactant such as fatty acid ethoxylate esters and ethers, sorbitane monoesters, and alkyl glycosides, ionic surfactants such as phophoslipids and alkyl sulphanates. In an embodiment of the invention the antimicrobial agent is against bacteria, virus, fungi, yeast or any other microorganism of the skin. Examples of microorganisms causing problems of the skin and especially present in wounds are *Staphylococcus aureus, Steptococcus pyrogenes, Staphylococcus pseudintermedius, Proprionibacterium acnes, Enterococcus faecalis, Bacillus subtilis, Pseudomonas aeuruginosa, Escherichia coli, Enterobacter coli, Malassezia Candida albicans* and *Aspergillus niger.* Said at least one antimicrobial agent is in an embodiment of the invention chosen from the group consisting of iodine solution, iodophors, hydrogen peroxide, chlorhexidine, acetic acid, honey, cetrimide, silver sulfadiazine, nanocrystalline silver and ionic silver. The at least one stabilizer is chosen depending on the antimicrobial agent used. A skilled person in the art knows which stabilizers that can be used.

The components are heated 10 - 15°C above the melting temperature of the pure components and mixed. In the case of the components used in this invention, they are heated to at least 70°C. The system has liquid crystalline lamellar structure at this temperature and the viscosity of the dispersion is low. The dispersion is then cooled quickly to the transition temperature between solid and liquid crystalline lamellar structure. In the case of the ingredients used in this invention, between about 30 and 37°C. After solidification of the crystals and increase of the viscosity of the composition, the temperature is decreased to the storage temperature, ≤ 25°C.

The composition according to the present invention is a cream (high viscous emulsion). A *thickener* chosen from the group consisting of polymers such as polysaccharides, polyacrylic, polyvinylic, polyvidone, polyethylene oxides, starch and cellulose polymers, and inorganic thickeners such as colloidal silica, bentonite, and saponite, can be added to adjust the viscosity. The main components of the wound healing composition are solvent and monoglycerides. The solvent, is in an embodiment of the invention preferably at least partly chosen from the humectants, to result in a improved moisturising composition. The monoglycerides are present in the composition at an amount of about 1-30 % by weight, and the solvent is present in an amount adding up to 100% by weight. The pH of the composition is 4-7.

The purity of the monoglyceride components are at least 80%, preferably 85%, with respect to monoglycerides. Monoglycerides are amphiphilic molecules. *Amphiphiles* are molecules that consist of segments with different preference regarding the solvent. One part of the amphiphile is hydrophilic (polar) and prefers contact with water or other hydrophilic compounds. The other part is hydrophobic (apolar) and prefers contact with oil or other hydrophobic compounds. *Monoglycerides* belong to the group natural low molecular weight amphiphiles. They are esters formed between fatty acids or fatty acid derivatives and glycerol and are called glycerides or glyceryl esters. There are a variety of glycerides used in food, cosmetic and drug products. Glyceryl esters are not pure monoesters but mixtures of mono-, di- and tri-esters. It also contains free glycerol and free fatty acid. The exact composition of the monoglycerides will depend on the source and the supplier of the material, because all commercially available reagents are not identical and the exact purity may vary depending on the manufacture process. A conventional composition used by industry to call the material monoester is that it should contain at least 80% monoester. The required purity for making the compositions in this invention is ≥ 80% monoester. The hydrocarbon chain length varies depending on the source. This invention covers hydrocarbon chain lengths from 8 to 14, where *Glyceryl caprylate* is the monoester of glycerol and caprylic acid (C8), *Glyceryl caprate* is the monoester of glycerol and capric acid (C10), *Glyceryl monolaurate* is the monoester of glycerol and lauric acid (C12), and *Glyceryl monomyristate* is the monoester of glycerol and myristic acid (C14).

Amphiliphilic molecules, such as monoglycerides, can spontaneously self-organize into microscopic structures in presence of solvent(s) due to the molecules amphilicity. Isotopic solutions are disordered over long and short distances but can form aggregates in one or two dimensions, so called micellar solutions. *Liquid crystalline structures* are ordered on long distances but disordered on short. The aggregates can be ordered in two dimensions (hexagonal and lamellar) or three dimensions (cubic). *Solid crystalline structures* are ordered both on short and long distances.

*Liquid crystalline lamellar structures* are formed by the monoglycerides used in this invention in combination with hydrophilic solvent and at elevated temperatures (> about 30°C). *Solid crystalline lamellar structures* are formed by the monoglycerides used in this invention in combination with hydrophilic solvent and at ambient temperatures (< about 30°C). Solid crystalline particles are dispersed in a continuous phase. The monoglycerides form a solid crystalline lamellar structure with rigid hydrocarbon chains tilted about 55° to the plane. The temperature when solid crystals are formed, when decreasing the product temperature during manufacture, is denoted *crystallisation temperature.* This temperature is visually determined in the examples below and should hence be regarded as a range around the noted temperature. The *melting temperature* is when a product is heated from its solid lamellar crystalline state to its liquid lamellar crystalline state. The melting temperatures given in the examples below are determined by drop point measurements. This is a standardised pharmacopeial method to determine the melting temperature. The melting temperature is in general higher compared to the crystallisation temperature for experimental set-ups on crystalline material.

The monoglycerides form the solid crystalline lamellar structure during manufacture when the temperature is decreased below the crystallisation temperature. One monoglyceride or a mixture of two or more monoglycerides, are mixed with solvent during manufacture to obtain a suitable crystallisation/melting temperature of the composition. The solid crystalline structure is kept during storage below the melting temperature.

A crystallisation temperature above about 30°C is preferred to have a composition with relatively high viscosity at storage at ambient temperatures. Above the crystallisation temperature of the composition, the viscosity is low and the product milk like. A crystallisation temperature between about 30 and 37°C is preferred if the composition contain an active pharmaceutical substance that should be released in contact with the body temperature but be encapsulated during storage at ambient temperatures.

The monoglyceride crystals are dispersed in a continous phase. The monoglycerides have preferably a hydrocarbon chain length of about C8 to C14 in this invention. The type of monoglycerides, amount and content ratio between the monoglycerides, the solvent and the manufacturing process, determines the crystal size and the viscosity of the composition as well as the crystallisation temperature and the moisturising capacity.

The composition act as a physical protective film ("dressing") and help control the level of moisture in a wound and thus reduces scab formation. The composition form a crystalline semi-occlusive dressing (Example 5) that has been shown to be optimal for wound healing (Example 1, Example 2, Example 3). The physical protective film formed by the composition, decrease the risk for mechanical trauma (Example 4), keep the body temperature, lower the pH, and help control the level of moist in a wound during the whole healing process, from the phase when the bleeding has stopped in an acute wound, a situation with excess moist, until the wound is closed and the scar building phase starts, with shortage of moist.

Moist is an important factor to control in wound healing. Directly after injury, an acute wound is wet; blood and other exudates needs to be absorbed. Later in the healing process a dry scab is formed if the wound is exposed to air. This scab containing dried exudates, blood or serum, acts as a barrier to body water loss. The scab replaces the normal protective barrier, the stratum corneum, which is missing in the injured area. If a scab is not formed it would result in significant body water loss. However, the reepithelialisation rate is decreased under a scab since cells and other relevant substances are hindered to diffuse. If a controlled moist environment can be kept during the whole healing process, skin growth is promoted, and scab formation reduced. The former has been solved with a composition of the present invention.

The composition according to present invention can absorb excess moist. Excess blood and other body fluids can be absorbed by the composition and the water holding capacity of the composition is large (Example 7). The excess water is absorbed between the monoglyceride crystals. The distance between the hydrophilic regions and the fat regions within the monoglyceride crystals vary only slightly with the water concentration and the temperature. From a film (barrier) forming perspective there is no difference between compositions containing liquid lamellar crystalline or solid lamellar crystalline structures around the skin temperature, about 30 - 37°C.

The composition of the present invention can deliver moist to a dry wound (Example 7). The activity of the water in the wound healing composition is high even if the structure of the composition is solid lamellar crystalline, there is free water available to moisturize a dry wound (Example 6). The formation of the film will maintain the moisture control with time through its water holding capacity, occlusion and humectant content.

Other key ingredients contribute to one or more of the following relevant functions listed in the inventory of the composition as a wound healing composition: skin conditioning, emollient, pH control, water retaining and antimicrobial. The principal intended action exploits the combined properties of these ingredients to assist the healing process; helping to restore skin condition, control the moisture balance, and normalise the microenvironment.

*Humectants* are substances that increase the hydration and the condition of the skin barrier, i.e. the stratum corneum (protect the skin from drying). Another model to explain the effect of humectants is to say that they preserve the fluidity of the lipids in the skin under dry conditions. Examples of humectants are glycerol, propylene glycol, butylene glycol, hexylene glycol, triacetin, panthenol, hyaluronic acid, pidolic acid, alfa-hydroxy acids, sorbitol, xylitol, manitol, and urea. Several of the humectants are naturally occurring in the skin. Examples of so called *natural moisturizing factors* (NMFs) in the skin are amino acids, lactates, citrates, sugars and inorganic salts. NMFs is a collection of water-soluble compounds that are found in the stratum corneum. All these can help to improve the skin barrier and can be included in the composition. Other skin conditioning substances such as vitamin B3 can be included to improve the product. Humectants, skin conditioning substances, and skin barrier improving substanses, are in the present context denoted together as humectants.

*Emollients* are materials that smoothen the surface of the skin and make the surface look uniform to the eye and silkier to touch. Emollients provide some occlusivity (Example 5). *Occlusive agents* increase moisture levels by providing a physical barrier to epidermal water loss. These two groups of substances are in the present context handled and denoted together as emollients. Examples of emollients are: *fat and vegetable oils -* subgroup of lipids including synthetic or natural glycerides (mono, di or triglyceride), *wax -* subgroup of lipids including natural or synthetic esters of fatty acids with long chain hydrocarbons, *silicones, petrolatum* (hard or soft paraffin) and *mineral oil* (liquid paraffin) - subgroups of lipids including non-vegetable long or short chain hydrocarbons (originating from petroleum).

*Water retaining* compounds can be added to increase the water binding capacity of the composition and make a physically stable composition during long term storage (several years at ambient temperatures without losing water). Examples of water retaining agents are *surfactants* - synthetic low molecular weight amphiphiles and high molecular weight *polymers* (Example 8). Examples of non-ionic surfactants are fatty acid ethoxylates esters and ethers, sorbitane monoesters and alkyl glucosides. Examples of ionic surfactants are phospholipids and alkyl sulphates. Examples of polymers are cellulose based polymers, polyacrylic acid and derivatives, and polysaccharides.

*Antimicrobial effect* is preferred to have from a wound healing composition. The composition in this invention is slightly antimicrobial due to the presence of glyceryl monolaurate (Example 9). The addition of an active pharmaceutical *antimicrobial agent* such as iodine solutions and iodophors, hydrogen peroxides, chlorhexidine, acetic acid, honey, cetrimide, silver sulfadiazine, etc. make the composition antimicrobial.

What was found out during development was that the composition without any active pharmaceutical antimicrobial ingredient was equally or even better in healing of wounds. It was surprisingly excellent as a moisturizing composition optimal for wound healing, especially suitable for dry wounds and not only open wounds and discharging skin areas as mentioned in US3772446.

The compositions developed have been shown to be excellent as wound healing compositions, even if they were developed to be drug delivery systems for difficult to stabilize active pharmaceutical substances such as hydrogen peroxide or as a general base for skin products. It has been shown in clinical trials that the composition without the hydrogen peroxide is better in healing of non-infected wounds compared to the composition with hydrogen peroxide (Example 1, Example 2). This surprisingly efficient wound healing effect is achieved by the composition of the present invention.

The wound healing progress is most rapid in an environment that is moist, warm, insulated and protected from mechanical trauma and bacterial invasion. The compositions in this invention form semi-occlusive moisture controlling protecting films in the wound during the whole healing process, from the phase when the bleeding has stopped in an acute wound, a situation with excess moist, until the wound is closed and the scar building phase starts, with shortage of moist. The compositions reduce also the risk for infection due to the low pH of the product and the antimicrobial effect from the monoglycerides. The risk for infection can be reduced even further if the composition contains hydrogen peroxide, which can be long term stabilised in the composition (several years of storage at ambient temperatures).

### Semi-occlusive moisture controlling composition

The antimicrobial effect of the monoglyceride composition containing hydrogen peroxide has been discussed extensively as well as the medical treatment of infections in wounds in presence of hydrogen peroxide or medical treatment to improve skin perfusion in presence of hydrogen peroxide. It was however surprising to see that the composition without the hydrogen peroxide was better in healing of non-infected wounds. Hence, the wound healing effect is related to the low pH of the composition, the presence of monoglycerides, and the moisture balancing control of the lipids and solvent in the composition.

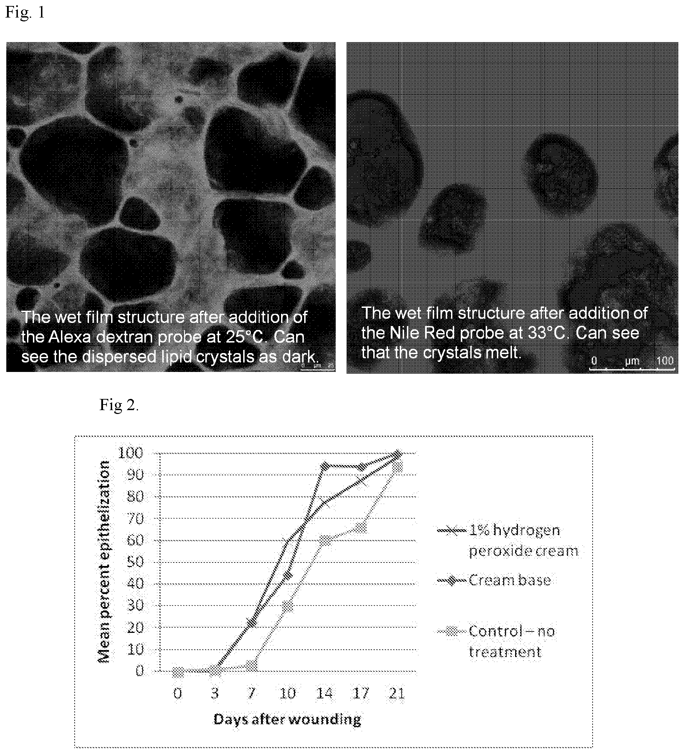

A lamellar crystalline structure has an advantage compared to a non-lamellar structure when it comes to keeping solvent within the structure. The swelling behaviour of the specific monoglycerides used has been known for a long time. The distance between the polar regions and the apolar regions within the crystals vary with temperature from room temperature up to about 80°C and solvent content from 0 to 100%. The size of the dispersed crystals depends on the cooling rate and the stirring during manufacture of the composition. The size with the manufacture process used in the examples in this invention are in the range 10-100 µm (Figure 1). The distance between the crystals depends on the amount of solvent in the system. The composition can stand accelerated forces and still keep the solvent within the structure (Example 8).The semi-occlusive film can swell and take up fluid from an exuding wound and moisturize a dry wound (Example 7). The activity of the water in the composition is high (Example 6).This means that even if the structure of the composition is solid lamellar crystalline there is free water available, and water can be absorbed or desorbed by the product. From a wound moisturizing perspective there is no difference between compositions containing liquid lamellar crystalline or solid lamellar crystalline structures. All these attributes are obtained by the composition itself without contribution of an active pharmaceutical substance such as hydrogen peroxide. Hydrogen peroxide has an ancillary action to the antimicrobial effect of the composition (Example 9). This effect is most important for infected wounds.

### Insulated and protected from mechanical trauma

The use of the wound healing composition according to the present invention instead of an occlusive dressing or in combination with a dressing, decrease the risk for disturbance of the wound healing process at redressing and thus mechanical trauma. The composition forms a soft protective film between the dressing and the wound surface. Without a dressing the composition will form a soft protective film to the environment without being greasy. The film is easily removed with water at redressing. The wound will be less sensitive to mechanical trauma by not drying and reducing scab formation and the wound will keep the body temperature when the composition is applied and the protective film is formed.

That the wound healing composition is decreasing the risk for wound healing disturbance at redressing was observed in a clinical investigation (Example 4). The monoglyceride composition was used as well as a comparative cream product. The comparative product was a traditional occlusive oil-in-water emulsion containing emollients. The active pharmaceutical substance in the comparator product was cetrimide, which is an antimicrobial substance. The study was undertaken in General Practitioners. The efficacy, side-effects and acceptability of the treatments on minor cuts, abrasions and burns was investigated. Both products showed excellent treatment in all indices of efficacy and the study was too small to significantly separate the treatment groups. No significant differences were found between the treatment groups in side-effects. The majority of the patients found the dressings easy to remove (97.7% in the crystalline monoglyceride cream group and 89.4% in the oil-in-water emulsion cream group). This difference was significant. The patients using the oil-in-water emulsion cream experienced that the dressing got stuck in the wound in significantly more cases compared to the group using the monoglyceride composition of the present invention. The same dressing was used for both groups.

### Protect from bacterial invasion

The risk for infection is a problem in wound healing. Wounds that are difficult to clean will contain microorganisms from the wounding trauma. Wounds that are not covered will, in a short time, be exposed to an environment of microorganisms. Some of these microorganisms present in a wound will significantly impair the healing process if they are allowed to grow in the wound and may eventually lead to infection.

Many wound healing products used for wound care have therefore some kind of antibacterial effect. Antiseptic solutions (iodine solutions and iodophors, hydrogen peroxides, chlorhexidine, ethanol etc) are often used to reduce the bacterial contaminations in a wound but may be negative for the wound healing process since they may affect the inflammation phase and the epithelialisation. Cleaning with water may therefore be recommended for cleaning of small cuts and wounds. Topical cream and ointments product with antimicrobial activity, such as topical antibiotics, povidone-iodine creams, silver sulfadiazine creams, are common to use. But the use of antibacterial products should be minimised due to for example the risk for bacterial resistance. A traditional treatment is to use honey on wounds. Honey has antibacterial activity due to for example the formation of hydrogen peroxide when diluted. Hydrogen peroxide is a well-known oxidizing, antiseptic agent that has been produced commercially since 1925. It has good antibacterial activity against both gram-positive and gram-negative bacteria and viruses and is used as an antiseptic, disinfectant and deodorant in pharmaceutical, cosmetic and food applications. Hydrogen peroxide is principally used as a chemical intermediate and for textile, paper and pulp bleaching as an oxidising agent. Large quantities are also used in industrial and municipal waste treatment. The property of hydrogen peroxide deemed most remarkable is that it readily decomposes into water and oxygen, and therefore poses absolutely no threat to the environment. It also decomposes rapidly to water and free oxygen in the presence of the endogenous enzyme catalase or peroxidase, i.e. in contact with body tissue. The antimicrobial activity of hydrogen peroxide depends on this fast release of oxygen when applied to tissue; the oxygen kills the bacteria. This is the reason for less risk for bacterial resistance to appear compared to many other antimicrobial agents. Hydrogen peroxide may theoretically affect the inflammation phase negatively in wound healing, as many other antimicrobial compounds, but it is not clear to what extent and it is less likely to appear at low concentration. The main problem to formulate hydrogen peroxide is to stabilise it at a low concentration in a product with long enough shelf-life. This was solved by incorporating the hydrogen peroxide into the lipid crystalline lamellar structure and the addition of a number of sequestrants, stabilizers and buffers. This has previously been described in US2002031556. The product is a low concentration slow release product and hence there is less risk for wound healing interference during the inflammation and epithelisation phase.

The use of glyceryl monolaurate makes it possible to formulate water based compositions without including traditional preservatives as glycerol monolaurate itself has antimicrobial effect (Example 9).

### Compositions

The composition according to the examples may be a lotion (low viscous emulsion), a cream (high viscous emulsion), a spray (very low viscous emulsion) or an ointment (water free system).

A crystallisation temperature above about 30°C is preferred, resulting in a composition with relatively high viscosity directly after manufacture and a product which viscosity that is not changing significantly during storage at ambient temperatures. Above the crystallisation temperature of the composition, the viscosity is low and the product milk like. A crystallisation temperature between about 30°C and 37°C is preferred if the product contain an active pharmaceutical substance that should be released in contact with the body temperature but be encapsulated during storage at ambient temperatures.

The monoglycerides have preferably a hydrocarbon chain length of about C8 to C14 in this invention. The type of monoglycerides, amount and content ratio between the monoglycerides, determines the crystal size and the viscosity of the composition as well as the crystallisation temperature. Preferred examples of a cream with optimal cream viscosity and glossy appearance, indicating a large crystal size which is not changing significantly during storage, are compositions made of C12 glyceryl monolaurate or C14 glyceryl monomyristate or a mixture of C12 glyceryl monolaurate and C14 glyceryl monomyristate at a ratio of 1:4 to 4:1 at a total amount 15 to 30% to form a cream (Composition 2, 3, 4, 6, and 7 in Table 1). The mixture of C8 glyceryl monocaprylate and C12 glyceryl monolaurate has a slightly too low viscosity (Composition 5 in Table 1). The viscosity is also too low when the amount of monoglycerides is <15% (Composition 1 in Table 1). The *drop point* is the temperature at which the first drop of the melting substance to be examined falls from a cup under defined conditions. The drop point is generally higher compared to the observed *crystallisation temperature* during manufacture. The crystallisation temperature, which is observed visually during manufacture, should be regarded as an interval which is dependent on the stirring rate and the cooling rate.

**Table 1**

| Composition | 1 | 2 | 3 | 4 | 5 | 6 | 7 |
|---|---|---|---|---|---|---|---|
| | Medium viscous emulsion | Cream | Cream | Cream | Low viscous emulsion | Cream | Cream |
| Component | % (w/w) | % (w/w) | % (w/w) | % (w/w) | % (w/w) | % (w/w) | % (w/w) |
| C14 Glyceryl monomyristate | 10 | 21 | 20 | 5 | | 28 | |
| C12 Glyceryl monolaurate | 3 | 7 | 5 | 20 | 20 | | 28 |
| C8 Glyceryl monocaprylate | | | | | 5 | | |
| Citric acid anhydrous | 0.9 | 0.9 | 0.9 | 0.9 | 0.9 | 0.9 | 0.9 |
| Sodium hydroxide | 0.3 | 0.3 | 0.3 | 0.3 | 0.3 | 0.3 | 0.3 |
| Purified water | 86 | 71 | 74 | 74 | 74 | 71 | 71 |
| Total | 100 | 100 | 100 | 100 | 100 | 100 | 100 |
| pH of product | 4 | 5 | 4 | 4 | 4 | 5 | 5 |
| Drop point of product | N/A | 41 | 41 | 35 | N/A | 47 | 38 |
| Crystallisation temperature | 32 | 36 | 32 | 30 | 30 | 37 | 34 |

| | | | | | | | |
|---|---|---|---|---|---|---|---|
| N/A not applicable | | | | | | | |

The viscosity is dependent on the amount of monoglycerides. The viscosity of a composition with low viscosity can be increased by addition of a thickener after or during manufacture. A *thickener* can be chosen from the group consisting of polymers such as polysaccharides, polyacrylic, polyvinylic, polyvidone, polyethylene oxides, starch and cellulose polymers, and inorganic thickeners such as colloidal silica, bentonite, and saponite. A cream is formed when the viscosity is relatively high (Composition 3, 4, 5 and 7 in **Table 2**).

**Table 2**

| | 1 | 2 | 3 | 4 | 5 | 6 | 7 |
|---|---|---|---|---|---|---|---|
| Composition | Very low viscous emulsion | Medium viscous emulsion | Cream | Cream | Cream | Low viscous emulsion | Cream |
| Component | %(w/w) | %(w/w) | %(w/w) | %(w/w) | %(w/w) | %(w/w) | %(w/w) |
| C14 Glyceryl monomyristate | 1 | 10 | 9 | 21 | 21 | | |
| C12 Glyceryl monolaurate | 1 | 3 | 3 | 7 | 7 | 20 | 20 |
| C8 Glyceryl monocaprylate | | | | | | 5 | 5 |
| Citric acid anhydrous | 0.9 | 0.9 | 0.8 | 0.9 | 0.9 | 0.9 | 0.9 |
| Sodium hydroxide | 0.3 | 0.3 | 0.3 | 0.3 | 0.3 | 0.3 | 0.3 |
| Glycerol | | | 9 | | | | |
| Carbomer (acrylic acid polymer) | | | 0.4 | | 0.3 | | 0.5 |
| Purified water | 97 | 86 | 78 | 71 | 71 | 74 | 73 |
| Total | 100 | 100 | 100 | 100 | 100 | 100 | 100 |
| pH of product | 5 | 4 | 4 | 5 | 5 | 4 | 4 |
| Drop point of product | N/A | N/A | 43 | 41 | 43 | N/A | n.m. |
| Crystallisation temperature | N/A | 32 | N/A | 36 | 31 | 30 | N/A |

| | | | | | | | |
|---|---|---|---|---|---|---|---|
| N/A not applicable if thickener added after manufacture or if not crytsallisations is not visible due to low viscosity, n.m. = not measured | | | | | | | |

The solvent is preferably water or a mixture of water and water soluble humectants, or water and emollients, to give a crystallisation temperature between 30 and 37°C. Composition 2, 4 and 5 in Table 3). Crystallisation temperature above 40°C is received if a solvent/humectant such as glycerol Composition 3 in Table 3) or an emollient such as rapeseed oil (Composition 1 in Tabl3 3) is used as solvent, and no water.

**Table 3**

| Composition | 1 | 2 | 3 | 4 | 5 |
|---|---|---|---|---|---|
| | Ointment | Cream | Ointment | Cream | Cream |
| Component | % (w/w) | % (w/w) | % (w/w) | % (w/w) | % (w/w) |
| C14 Glyceryl monomyristate | 15 | 21 | 10.5 | 21 | 21 |
| C12 Glyceryl monolaurate | 5 | 7 | 3.5 | 7 | 7 |
| Citric acid anhydrous | | 0.9 | 0.9 | 0.9 | 0.9 |
| Sodium hydroxide | | 0.3 | 0.3 | 0.3 | 0.3 |
| Glycerol | | | 85 | 20 | |
| Rapeseed oil | 80 | 20 | | | |
| Purified water | | 51 | | 51 | 71 |
| Total | 100 | 100 | 100 | 100 | 100 |
| pH of product | N/A | 4 | 5 | 5 | 5 |
| Drop point of product | 54 | 42 | 51 | 41 | 41 |
| Crystallisation temperature | 47 | 36 | 47 | 34 | 36 |

| | | | | | |
|---|---|---|---|---|---|
| N/A not applicable | | | | | |

The composition may also contain water retaining compounds to increase the long term stability and avoid separation of water. The drop point and the viscosity is minimally affected. In this example PEG-100 stearate and PEG-20 stearyl ether were used as surfactant and Carbomer homopolymer type A (polyacrylic acid) and hydroxyethyl cellulose as polymers to increase the water retention. Other types of cosmetically or pharmaceutically acceptable non-ionic and ionic surfactants and non-ionic and ionic polymers can be used obtaining the same physical stability of the composition.

**Table 4**

| Composition | 1 | 2 | 3 | 4 | 5 | 6 | 7 |
|---|---|---|---|---|---|---|---|
| | Cream | Cream | Cream | Cream | Cream | Cream | Cream |
| Component | % (w/w) | % (w/w) | % (w/w) | % (w/w) | % (w/w) | % (w/w) | % (w/w) |
| C14 Glyceryl monomyristate | 21 | 21 | 21 | 21 | 21 | 21 | 21 |
| C12 Glyceryl monolaurate | 7 | 7 | 7 | 7 | 7 | 7 | 7 |
| Citric acid anhydrous | 0.9 | 0.9 | 0.9 | 0.9 | 0.9 | 0.9 | 0.9 |
| Sodium hydroxide | 0.3 | 0.3 | 0.3 | 0.3 | 0.3 | 0.3 | 0.3 |
| Propylene glycol | | | | 2 | | | |
| Glycerol | | | | | 5 | | |
| PEG-100 stearate | | 1 | | 1 | 1 | | |
| PEG-20 stearyl ether | | | | | | 1 | |
| Carbomer (polyacrylic acid) | | | 0.3 | | | | |
| Hydroxyethyl cellulose | | | | | | | 0.4 |
| Purified water | 71 | 70 | 71 | 68 | 65 | 70 | 70 |
| Total | 100 | 100 | 100 | 100 | 100 | 100 | 100 |
| pH of product | 5 | 4 | 5 | 5 | 5 | n.m. | n.m. |
| Drop point of product | 41 | 40 | 43 | 43 | 42 | n.m. | n.m. |
| Crystallisation temperature | 36 | 37 | 31 | 36 | 37 | 37 | 35 |
| Centrifugation (10000 rpm, 5 min, 20°C) | separation of water | No separation | No separation | No separation | No separation | indication of separation | slight indication of separation |

| | | | | | | | |
|---|---|---|---|---|---|---|---|
| N/A not applicable, n.m. = not measured | | | | | | | |

The composition may also contain humectants such as glycerol, propylene glycol, urea, lactic acid and other types of skin conditioning compounds acceptable to use in cosmetic and/or pharmaceutical products. *Humectants* are substances that increase the water-binding capacity of the skin, the skin barrier, i.e. the stratum corneum. Another model to explain the effect of humectants is to say that they preserve the fluidity of the lipids in the skin under dry conditions.

**Table 5**

| Composition | 1 | 2 | 3 | 4 | 5 | 6 | 7 |
|---|---|---|---|---|---|---|---|
| | Cream | Cream | Cream | Cream | Cream | Cream | Cream |
| Component | % (w/w) | % (w/w) | % (w/w) | % (w/w) | % (w/w) | % (w/w) | % (w/w) |
| C14 Glyceryl monomyristate | 10.5 | 21 | 21 | 21 | 21 | 21 | 21 |
| C12 Glyceryl monolaurate | 3.5 | 7 | 7 | 7 | 7 | 7 | 7 |
| Citric acid anhydrous | 0.9 | 0.9 | 0.9 | 0.9 | | 1 | 0.9 |
| Sodium hydroxide | 0.3 | 0.3 | 0.3 | 0.3 | 0.4 | 0.4 | 0.3 |
| Propylene glycol | | | 2 | | | | |
| Glycerol | 85 | 20 | | 5 | | | |
| Lactic acid | | | | | 5 | | |
| Carbamide (urea) | | | | | | 10 | |
| Niacinamide (vitamin B3) | | | | | | | 5 |
| PEG-100 stearate | | | 1 | 1 | | | |
| Carbomer | | | | | | 0.2 | |
| Purified water | | 51 | 68 | 65 | 67 | 60 | 54 |
| Total | 100 | 100 | 100 | 100 | 100 | 100 | 100 |
| pH of product | 5 | 5 | 5 | 5 | 5 | 5 | 5 |
| Drop point of product | 51 | 41 | 43 | 42 | 41 | n.m. | n.m. |
| Crystallisation temperature | 47 | 34 | 36 | 37 | 34 | <40 | 47 |

| | | | | | | | |
|---|---|---|---|---|---|---|---|
| n.m. = not measured | | | | | | | |

All compositions contain agents to obtain a product pH of 4-7, which is in the range of normal skin pH. pH in this region is preferred in a product to be applied in wound and on skin in general.

Antimicrobial agents such as hydrogen peroxide and stabilisers to increase the long term stability of hydrogen peroxide can be added. The formulation containing 1-3% hydrogen peroxide has a crystallisation temperature and viscosity in the same region as samples without hydrogen peroxide. Hydrogen peroxide specific stabilisers do not affect the crystallisation temperature and the viscosity significantly.

**Table 6**

| Composition | 1 | 2 | 3 |
|---|---|---|---|
| | Cream | Cream | Cream |
| Component | % (w/w) | % (w/w) | % (w/w) |
| C14 Glyceryl monomyristate | 21 | 21 | 21 |
| C12 Glyceryl monolaurate | 7 | 7 | 7 |
| Citric acid anhydrous | 0.9 | 0.9 | 0.9 |
| Sodium hydroxide | 0.3 | 0.3 | 0.3 |
| Propylene glycol | | 2 | 2 |
| Hydrogen peroxide | | 1 | 1 |
| Stabilisers (salicylic acid, sodium stannate, sodium oxalate, EDTA, sulphuric acid, sodium pyrophosphate) | | | 0.4 |
| Purified water | 71 | 68 | 67 |
| Total | 100 | 100 | 100 |
| pH of product | 5 | 4 | 5 |
| Drop point of product | 41 | 42 | n.m. |
| Crystallisation temperature | 36 | 37 | <40 |

| | | | |
|---|---|---|---|
| n.m. = not measured | | | |

### Examples

### Example 1. Clinical effect in humans

A randomised, double blind study evaluated the wound healing capabilities of monglyceride compositions (a cream) with propylene glycol as humectant, with and without hydrogen peroxide, on the skin of eight healthy volunteers aged 21 to 40. Subjects received twice daily application of the study treatments to wounds on their forearms. Three distinct areas of the forearm where marked and wounded using a Shelanski dermatome. Once haemostasis had been achieved, the study treatments (with and without hydrogen peroxide) were applied to two of the three marked test sites according to the randomisation scheme. The third area received no treatment; serving as a control. Treated and untreated wound sites were covered with a dressing and subjects were asked to keep the sites dry. For the next 21 days, the test compositions were reapplied each morning and evening and again covered with a dressing. Visual examinations and estimation of epithelialisation were made on the evaluation days 4, 8, 12, 15, 18 and 22 (3, 7, 11, 14, 17 and 21 after wounding day). All evaluations were blindly performed by an individual other than the one administering the treatments.

Each wound site was examined via a stereo microscope and estimation (to the nearest 10%) of epithelialisation was made. The investigator also ranked each of the wound sites clinically as 1=the best, 2= middle, 3= the worst. This evaluation considered the general appearance of the wound, the degree of redness or inflammation and swelling if present. A photograph of each wound was taken at all evaluation times. All evaluations were blindly performed by an individual other than the one administering the treatments.

On all evaluation days, statistical analysis of the rate of reepithelialisation demonstrated superiority of the monoglyceride compositions (with and without hydrogen peroxide) in comparison to no treatment; this superiority was significant on day 7 and onwards (Figure 2). On days 14 and 17, wounds treated with the composition without hydrogen peroxide appeared sinficantly clinically better than those treated with hydrogen peroxide. This shows that improved moisture controlled healing, protection agains mechanical trauma and reducing scab formation was achieved by the monoglyceride cream.

### Example 2. Clinical effect in pigs

A single blinded study was performed to evaluate the effect of monoglyceride compositions (a cream), with propylene glycol as humectant, on the healing of partial thickness wounds in domestic pigs. Three young Yorkshire pigs (between 5.5 and 9.0kg) were housed in an animal facility during the study. Each animal was clipped using a standard clipper and the skin was washed. Between 90 and 130 rectangular wounds (7x10 mm, and 0.3 mm deep) were made in the paravertebal and thoracic area using an electrokeratome. The wounds were separated from one another by at least 15 mm of normal skin. The wounds were divided into three equal treatment groups (about 35 wounds in each group). One wound group was treated with monoglyceride cream, another with monoglyceride cream with 1% hydrogen peroxide, about 5 minutes after surgery. The third wound was left without treatment and served as an untreated control.

Each day post-wounding beginning on day 2, the several wound were excised using the electrokeratome, set at a slightly deeper setting compared to the initial wound and evaluated for epidermal resurfacing after first removing the dermis by 2N sodium bromide treatment. If a visible hole in the epidermis was observed, the wound was countered as not healed. By evaluating wounds from each treatment group every day, the percent of healed wounds could be determined each day.

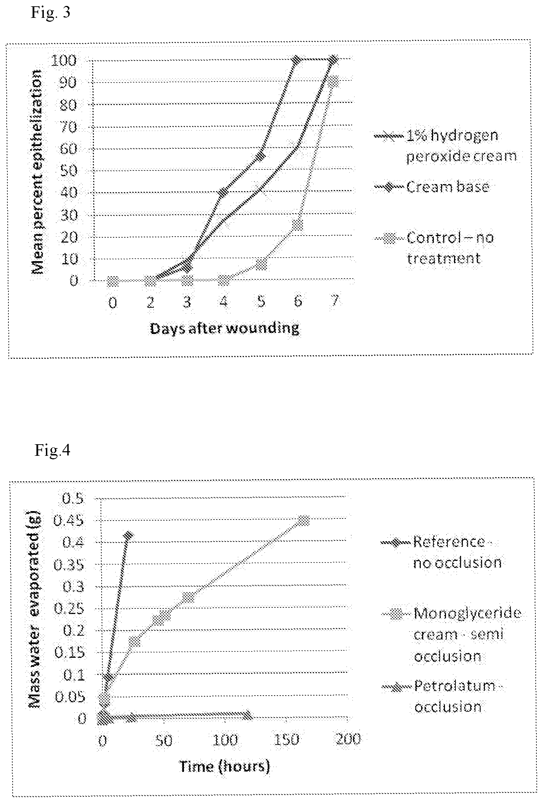

Both compositions, with and without hydrogen peroxide, enhanced the healing compared to untreated wounds. The composition without hydrogen peroxide accelerated the healing most (Figure 3). No adverse events were recorded. This shows that improved moisture controlled healing was achieved by the monoglyceride cream.

### Example 3. Clinical effect in horses

Two studies were performed on adult, healthy, non-pregnant standard breed mares and geldings. The horses were kept under identical housing conditions indoors and turned out on the same paddock during daytime during the study.

In the first study three areas were shaved on the neck of the horses and full-thickness wounds were created at the centre of each shaved area with a 2 cm diameter punch by the same surgeon on day 1. In the second study two areas were shaved on the neck of the horses and full-thickness wounds were created at the centre of each shaved area with a 2 cm diameter punch by the same surgeon on day 1. Each wound was uniformly cleaned with three swabs soaked with sterile 0.9% sodium chloride twice daily. The wounds were randomly treated with monoglyceride composition (a cream) ,with propylene glycol as humectant, with and without hydrogen peroxide, non-treated and treated with petrolatum ointment. The wounds were unprotected and left to heal by second-intention. All wounds were assessed daily until complete epithelisation. The wound was considered healed when an epithelial layer covered the entire wound surface.

The horses were examined daily for general status, fever, unusual behaviour, and indication of wound infection. The wounds were evaluated for protocol data on day 2, 6, 11, 16, 21 and 28. A scoring system for swelling, sensitivity to touch, discharge, granulation and epithelisation was used for subjective evaluation. Bacterial cultures and sterile swabs for cytology were thereafter taken from all wounds. Bacterial cultures were obtained on day 2, 6 and 16. Finally the wounds were cleaned using the procedure described above and the wounds were photographed. After day 28 the unhealed wounds were ocularly examined daily.

Both monoglyceride compositions appeared safe to use and effective for topical wound treatment or wound protection. The healing was significantly faster compared to untreated wounds and petrolatum treated wounds (occluded wounds). This shows that improved moisture controlled healing, protection agains mechanical trauma and reducing of scab formation was achieved by the monoglyceride compositions.

### Example 4. Clinical acceptability

An open comparator study was undertaken in General Practice (multi-centre study, 19 sites) to determine the efficacy, side-effects and acceptability of a monoglyceride composition (a cream), with propylene glycol as humectant, containing 1% hydrogen peroxide in the treatment of minor cuts, abrasions and burns. The comparator product was Cetavlex cream (cetramide 0.5%w/w) which is a well-established product for the treatment of minor wounds, burns and abrasions.

The time to complete healing was the principal criterion of satisfactory medication. An estimate of drug efficacy (0-100%) was made and a description of adverse events was recorded. Observations whether the dressings adhere to the wound surface and whether they are able to be removed easily without sticking were observed.

Patients between 7 and 75 years of age presenting for the first time with a recent minor injury - minor cuts, abrasions or second degree burns - were eligible for enrolment into the trial. Median time since injury to presentation was 24 hours, ranging from a few minutes to 30 days. Patients requiring sutures, with abrasions exceeding 25 cm² in area or with burns requiring admission to hospital, were not eligible. Patients were randomised to monoglyceride composition(a cream) or Cetavlex cream.

The skin site was swabbed, treated with the allocated cream and covered with a non-medical non-sticking dressing at the General Practitioner. This procedure was then repeated by the patient twice daily.

The study demonstrated that the efficacy of the monoglyceride composition and Cetalvex cream in the treatment of minor injuries were not clinically significantly different. The healing was good in both groups. The study was too small to separate the groups with regard to efficacy. No side-effects were reported from the monoglyceride composition. One patient in the Cetavlex group experienced mild-burning for the initial two days of the study following application of cream. There were no withdrawals from the study due to side-effects. At the end of the trial, patients were asked if the dressings were easy to remove (i.e. without sticking). The majority of the patients found the dressings easy to remove, 97.7% in the monoglyceride composition group and 89.4% in the Cetavlex group. This difference was significant.

The conclusion from the study was that the monoglyceride cream was well accepted by the patient, showed excellent healing and that it decreased the risk for mechanical trauma at redressing. This shows that improved moisture controlled healing, protection agains mechanical trauma and reducing scab formation was achieved by the monoglyceride cream.

| | **Monoglyceride composition** | | **Cetavlex cream** | |
|---|---|---|---|---|
| **Was the dressing easy to remove?** | **No of patients** | **%** | **No of patients** | **%** |
| No | 2 | 2.3 | 9 | 10.6 |
| Yes | 86 | 97.7 | 76 | 89.4 |
| Total | 88 | 100 | 85 | 100 |

### Example 5. Semi-occlusion

The occlusive effect was measured by making evaporation experiments. Thin film of a composition (a cream), with propylen glycol as humectant, of the invention was prepared and the possibility of water to evaporate was measured in weight.

Thin films of a composition were prepared in a PE-test tube with a PE-lid, tightened with PE-film to avoid evaporation of water outside the film. Two stainless steel nets, placed above each other, were placed on the inside of the lid and used as composition support. A thin film of composition was applied in the stainless steel support. The film thickness was < 1 mm. The test cell was filled with water up to about 5 mm from the stainless steel support. This would correspond to 100% RH below the composition film. The humidity in the room was about 10-20% RH during the experiment and the temperature about 23°C.

The cream decreases the evaporation rate but water evaporate slowly through the film, i.e., the composition is semi-occlusive (Figure 4). Petrolatum was occlusive and limited water could penetrate through the film.

### Example 6. Water activity

The water activity was measured on a composition (a cream) on a hygroscope (Rotronic Hygroskop DT with the humidity sensor DMS 100H). The experiment was performed at about 22°C, when the composition was in solid crystalline structure. The measurements were made in duplicate. The activity of the water in the composition was 94.8 and 94.5% RH, respectively. A water activity of 95% RH shows that most of the water is "free" water, i.e. not bound water, and it can diffuse freely.

The composition (a cream) of the present invention can deliver moist to a dry wound. The formation of the protective film will maintain the moisture control with time through occlusion.

### Example 7. Water absorption/desorption

The absorption of water by a composition (a cream) was investigated by making simple visual experiments. Monoglyceride cream was spread onto a glass plate. Water dyed with methylene blue was added to demonstrate that the cream has an ability to absorb and hold excess water. The experiment was repeated after first evaporating about 50% of the water from the monoglyceride cream. Also this cream had the same ability to reuptake water after evaporation. To illustrate the behaviour where water is not taken up, the experiment was repeated using white soft paraffin, which makes a completely occlusive film. A glass plate was smeared with paraffin and dyed water was added. The water forms a drop on top of the film.

### Example 8. Water retaining

The monoglyceride composition (a cream) can contain water retaining compounds and thickeners to increase the long term stability and avoid separation of water. In this example PEG-100 stearate and PEG-20 stearyl ether were used as surfactant and Carbomer homopolymer type A (polyacrylic acid) and hydroxyethyl cellulose as thickener to increase the water retention. The samples were centrifuged. The samples were centrifuged for 5 minutes at 10 000 rpm at 20°C, .i.e., the samples were in the solid crystalline storage state. The composition without any water retaining compound phase separated after centrifugation. Water was seen as a supernatant phase. The sample with PEG-20 stearyl ether showed an indication for phase separation and the sample with hydroethyl cellulose showed a slight indication for phase separation. The remaining samples were visually not affected by the centrifugation.

### Example 9. Antimicrobial effect

The compositions have an inbuilt antimicrobial system which gives less need for preservatives. The antimicrobial activity of the composition is partly due to the presence of hydrogen peroxide and partly due to the presence of glyceryl monolaurate. Glyceryl monolaurate is antimicrobially active against bacteria (e.g. *S*. *aureus, B. subtilis, Pseud. aeuruginosa*)*.* In combination with hydrogen peroxide, a synergistic effect is achieved and antimicrobial activity according to A criteria is obtained against also yeast (e.g. *Candida albicans*) and molds (*Aspergillus niger*)*.* This was shown by performing a preservative effectiveness test according to the PhEur method. Acceptance criteria A is the most strict criteria according to PhEur. Criteria B is less strct.

| | | Cream containing H₂O₂ and stabilisers | Cream containing stabilisers but not H₂O₂ | Cream base |
|---|---|---|---|---|
| *Candida albicans* | Fungi | A | A | B |
| *Aspegillus brasiliensis* | Fungi | A | B | A |
| *Pseudomonas aeruginosa* | Bacteria | A | A | A |
| *Staphylicoccus aureus* | Bacteria | A | A | A |

The invention is shown by Composition 4 of table 3, Compositions 4 and 5 of table 4, Composition 2-7 of table 5, and Compositions 2-3 of table 6. All Compositions of tables 1 and 2, Compositions 1-3, 5 in table 3, Compositions 1-3, 6-7, in table 4, Composition 1 in table 5, and Composition 1 in table 6, shall be considered as comparative examples of the Composition.

## Claims

1. A composition comprising glyceryl monolaurate and glyceryl monomyristate, and at least one hydrophilic solvent, having a liquid crystalline lamellar structure at a temperature of above about 37°C, a solid crystalline lamellar structure below about 30°C and is in transformation of said solid and liquid crystalline lamellar structures at from about 30°C to about 37°C, a humectant, and optionally one or more of a stabilizer, a preservative, an emollient, a buffering agent, a water retaining compound, or an antimicrobial agent, for use in a method for promoting healing in connection with injuries and wounds in mammals, wherein said composition being a cream, and wherein the monoglycerides comprise a mixture of C12 glycerol monolaurate and C14 glyceryl monomyristate at a ratio of 1:4 to 4:1 at a total amount 15 to 30 % by weight, and the solvent is present in an amount adding up to 100% by weight.

2. A composition for use according to claim 1, wherein said injuries and wounds are abrasions, cuts, burns, lacerations, blisters, acne spots, bites, rash wounds, chronic wounds including venous and diabetic leg and foot ulcers, ulcers caused by poor blood circulation, or wounds caused by eczema or infections.

3. A composition for use according to claim 1, wherein said humectant is chosen from the group consisting of glycerol, propylene glycol, butylene glycol, hexylene glycol, triacetin, panthenol, pidolic acid, alfa-hydroxy acids, sorbitol, xylitol, manitol, vitamin B3 and urea.

4. A composition for use according to claim 1, wherein said emollient is chosen from the group consisting of fat and vegetable oil, wax, petrolatum, hard or soft paraffin, silicone and mineral oil.

5. A composition for use according to claim 1, wherein said water retaining compound is chosen from the group consisting of surfactants and polymers, cellulose based polymers, polyacrylic acid derivatives, polysaccharides, non-ionic surfactant such as fatty acid ethoxylate esters and ethers, sorbitane monoesters, ionic surfactants such as phophoslipids and alkyl sulphanates.

6. A composition for use according to claim 1, wherein said antimicrobial agent is against bacteria, fungi, virus, yeast or any other microorganism of the skin.

7. A composition for use according to claim 6 wherein said at least one antimicrobial agent is chosen from the group consisting of iodine solution, iodophors, hydrogen peroxide, chlorhexidine, acetic acid, cetrimide, and silver.

8. A composition for use according to anyone of claims 1-7, wherein the purity of the composition is at least 80%, preferably 85%, with respect to monoglycerides.

9. A composition for use according to anyone of claims 1-8, wherein the pH of the composition is 4-7.

## Patentansprüche

1. Zusammensetzung, umfassend Glycerylmonolaurat und Glycerylmonomyristat und mindestens ein hydrophiles Lösungsmittel mit einer flüssigkristallinen lamellaren Struktur bei einer Temperatur oberhalb von etwa 37 °C, einer festkristallinen lamellaren Struktur unterhalb von etwa 30 °C und im Übergang der fest- und flüssigkristallinen laminaren Struktur bei etwa 30 °C bis etwa 37 °C, ein Feuchthaltemittel und gegebenenfalls einen Stabilisator, einen Konservierungsstoff, ein Emolliens, ein Puffermittel, eine Wasser zurückhaltende Verbindung und/oder ein antimikrobielles Mittel, zur Verwendung bei einem Verfahren zur Förderung der Heilung in Verbindung mit Verletzungen und Wunden bei Säugetieren, wobei es sich bei der Zusammensetzung um eine Creme handelt und wobei die Monoglyceride eine Mischung von C12-Glycerylmonolaurat und C14-Glycerylmonomyristat in einem Verhältnis von 1:4 bis 4:1 in einer Gesamtmenge von 15 bis 30 Gew.-% umfassen und das Lösungsmittel in einer Menge vorliegt, die sich zu 100 Gew.-% summiert.

2. Zusammensetzung zur Verwendung nach Anspruch 1, wobei es sich bei den Verletzungen und Wunden um Abschürfungen, Schnitte, Verbrennungen, Lazerationen, Blasen, Akneflecken, Bisse, Ausschlagwunden, chronische Wunden einschließlich venösen und diabetischen Bein- und Fußgeschwüren, durch schlechte Blutzirkulation verursachte Geschwüre oder durch Ekzem oder Infektionen verursacht Wunden handelt.

3. Zusammensetzung zur Verwendung nach Anspruch 1, wobei das Feuchthaltemittel aus der Gruppe bestehend aus Glycerin, Propylenglykol, Butylenglykol, Hexylenglykol, Triacetin, Panthenol, Pidolsäure, alpha-Hydroxysäuren, Sorbitol, Xylitol, Mannitol, Vitamin B3 und Harnstoff ausgewählt ist.

4. Zusammensetzung zur Verwendung nach Anspruch 1, wobei das Emolliens aus der Gruppe bestehend aus Fett und Pflanzenöl, Wachs, Petrolatum, Hart- oder Weichparaffin, Silikon und Mineralöl ausgewählt ist.

5. Zusammensetzung zur Verwendung nach Anspruch 1, wobei die Wasser zurückhaltende Verbindung aus der Gruppe bestehend aus Tensiden und Polymeren, Polymeren auf Cellulosebasis, Polyacrylsäurederivaten, Polysacchariden, nichtionischen Tensiden wie Fettsäureethoxylatestern und -ethern, Sorbitanmonoestern, ionischen Tensiden wie Phospholipiden und Alkylsulfanaten ausgewählt ist.

6. Zusammensetzung zur Verwendung nach Anspruch 1, wobei das antimikrobielle Mittel gegen Bakterien, Pilze, Viren, Hefen oder einen beliebigen anderen Mikroorganismus der Haut ist.

7. Zusammensetzung zur Verwendung nach Anspruch 6, wobei das mindestens eine antimikrobielle Mittel aus der Gruppe bestehend aus Iodlösung, Iodophoren, Wasserstoffperoxid, Chlorhexidin, Essigsäure, Cetrimid und Silber ausgewählt ist.

8. Zusammensetzung zur Verwendung nach einem der Ansprüche 1-7, wobei die Reinheit der Zusammensetzung, bezogen auf Monoglyceriden, mindestens 80 %, vorzugsweise 85 %, beträgt.

9. Zusammensetzung zur Verwendung nach einem der Ansprüche 1-8, wobei der pH-Wert der Zusammensetzung 4-7 beträgt.

## Revendications

1. Composition comprenant du monolaurate de glycéryle et du monomyristate de glycéryle, et au moins un solvant hydrophile, possédant une structure lamellaire cristalline liquide à une température supérieure à 37 °C, une structure lamellaire cristalline solide en dessous d'environ 30 °C et étant en transformation entre lesdites structures lamellaires cristallines solides et liquides à une température d'environ 30 °C à environ 37 °C, un humectant, et éventuellement un ou plusieurs parmi un agent stabilisant, un conservateur, un émollient, un agent tampon, un composé pour la rétention d'eau, et un agent antimicrobien, pour une utilisation dans un procédé pour la promotion de la cicatrisation en relation avec des lésions et des blessures chez des mammifères, ladite composition étant une crème, et les monoglycérides comprenant un mélange de monolaurate de glycérol en C12 et de monomyristate de glycéryle en C14 en un rapport de 1:4 à 4:1 à raison d'une quantité totale de 15 à 30 % en poids, et le solvant étant présent en une quantité s'additionnant jusqu'à 100 % en poids.

2. Composition pour une utilisation selon la revendication 1, lesdites lésions et blessures étant des abrasions, des coupures, des brûlures, des lacérations, des cloques, des boutons d'acné, des morsures, des éruptions cutanées, des blessures chroniques y compris des ulcères de la jambe et du pied veineux et diabétiques, des ulcères causés par une mauvaise circulation sanguine, ou des blessures causées par de l'eczéma ou des infections.

3. Composition pour une utilisation selon la revendication 1, ledit humectant étant choisi dans le groupe constitué par le glycérol, le propylèneglycol, le butylèneglycol, l'hexylèneglycol, la triacétine, le panthénol, l'acide pidolique, des alpha-hydroxyacides, le sorbitol, le xylitol, le mannitol, la vitamine B3 et l'urée.

4. Composition pour une utilisation selon la revendication 1, ledit émollient étant choisi dans le groupe constitué par une graisse et une huile végétale, une cire, un pétrolatum, une paraffine dure ou molle, une silicone et une huile minérale.

5. Composition pour une utilisation selon la revendication 1, ledit composé pour la rétention d'eau étant choisi dans le groupe constitué par des tensioactifs et des polymères, des polymères à base de cellulose, des dérivés de poly(acide acrylique), des polysaccharides, un tensioactif non ionique tel que des esters et des éthers d'éthoxylate d'acide gras, des monoesters de sorbitane, des tensioactifs ioniques tels que des phospholipides et des sulfanates d'alkyle.

6. Composition pour une utilisation selon la revendication 1, ledit agent antimicrobien étant contre des bactéries, des champignons, des virus, des levures ou un quelconque autre micro-organisme de la peau.

7. Composition pour une utilisation selon la revendication 6, ledit au moins un agent antimicrobien étant choisi dans le groupe constitué par une solution d'iode, des iodophores, le peroxyde d'hydrogène, la chlorhexidine, l'acide acétique, le cétrimide, et l'argent.

8. Composition pour une utilisation selon l'une quelconque des revendications 1 à 7, la pureté de la composition étant d'au moins 80 %, préférablement 85 %, par rapport aux monoglycérides.

9. Composition pour une utilisation selon l'une quelconque des revendications 1 à 8, le pH de la composition étant de 4 à 7.
